# EUROPEAN PATENT SPECIFICATION

(11) **EP 2 074 391 B1**
(45) Date of publication and mention of the grant of the patent: **11.02.2015**
(21) Application number: 07852507.8
(22) Date of filing: 03.10.2007
(51) Int. Cl.: G01F 23/292, A61L 9/04, A61L 9/12, G01N 19/10, H04Q 9/00

(54) **FRAGRANCE DEVICE WITH FRAGRANCE AMOUNT INDICATOR**
DUFTEINRICHTUNG MIT DUFTMENGENINDIKATOR
DIFFUSEUR DE PARFUM AVEC INDICATEUR DE CONSOMMATION

(30) Priority: 03.10.2006 US 848962 P
(43) Date of publication of application: 01.07.2009
(73) Proprietor: Scent2Market Inc., Yonkers, NY 10701-6806 (US)
(72) Inventor: D'AMICO, Daniel, South Salem, NY 10590 (US)
(74) Representative: Baumbach, Friedrich
(86) International application number: PCT/US2007/021228
(87) International publication number: WO 2008/042405

(56) References cited:
- WO-A1-92/15338
- WO-A1-2004/043502
- WO-A2-2005/003821
- US-A- 5 647 052
- US-A- 6 039 212
- US-A1- 2003 188 377
- US-B1- 6 227 458
- US-B1- 6 390 453

## Description

### Claim to Priority

This application claims priority to United States Provisional Patent Application No. 60/848,962, filed on October 3, 2006, and having a common inventor as this application. That application is incorporated by reference herein as if rewritten in its entirety.

### Background of the Invention

### 1. Field of the Invention

The invention relates to the fields of scent and aroma management. This may include but not be limited to methods and apparatuses used to notify a user that the contents of a fragrance dispenser have exhausted or will soon be exhausted.

### 2. Background

Many devices for providing a fragrance to an area over a period of time are known. Generally, these devices operate by allowing a fragrance contained in the device to diffuse from the device and into the atmosphere. Diffusion of fragrance may be enhanced, for example, by heating the fragrance, by including the fragrance in a volatile carrier, or by a combination of those. Diffusion of fragrances and their general intensity is governed by their equilibrium vapor pressure. Fragrances with a high equilibrium vapor pressure have a high volatility and quickly evaporate.

The useful lifespan of a fragrance device is limited by the amount of fragrance that is able to be distributed from the device. After the fragrance supply of the device is exhausted, either the device is replaced in its entirety, or the fragrance is replaced. This latter may be done, for example, by replacement of a removable cartridge in the fragrance device.

Unfortunately, determining when the fragrance must be replaced is an arduous task, particularly when a large number of fragrance devices are in use simultaneously, as in an office building or other large facility. Visual inspection of the fragrance amount may be inaccurate or impossible. Scheduled replacement of fragrance may be wasteful or ineffective, since fragrances may not be exhausted at a uniform rate over time or throughout a facility. This could result in waste of fragrance or in a period of time during which fragrance is not available. Variable exhaustion may be caused by differences in atmospheric conditions or size of an area to be filled with fragrance.

It would be useful for a user of a fragrance device to receive notice when the fragrance device has exhausted its supply of fragrance. It would further be useful for the user of a fragrance device to receive a warning when the amount of fragrance remaining in a device reaches a predetermined level.

### Brief Summary of the Invention

In one aspect, the invention includes an fragrance-dispensing device that provides a signal to a user of the device. This signal may indicate, for example, that the fragrance in the device has been exhausted. The signal may indicate that the amount of fragrance in the device has reached or fallen below a predetermined amount. A single device may provide more than one signal, so that the status of the fragrance in the device may be continually assessed. This may include providing a signal when the amount of fragrance has decreased or has been entirely exhausted.

In one embodiment, the fragrance is contained in a carrier. The carrier may be, for example, a polymer. The polymer may be, for example, ethyl vinyl acetate. The polymer will shrink as the fragrance is exhausted. After the polymer has shrunk to a certain level, a signal is actuated to indicate that the amount of fragrance in the device has been reduced to a predetermined amount or has been entirely exhausted. Additional signals may be actuated as the fragrance continues to be exhausted and the polymer continues to shrink. The signal may be, for example, a radio signal, Bluetooth signal, electrical signal, wi-fi signal, a light, a sound, a switch, or any other notification signal or combination thereof. In some embodiments the signal may be continuously updated to give a real-time assessment of the amount of fragrance that remains.

In a further embodiment A system for managing the fragrance amounts in a plurality of fragrance dispensers is provided, comprising providing a plurality of fragrance dispensers according to the invention; assigning each fragrance dispenser a separate identifying value that is transmitted with the signal; maintaining a database of identifying values for each fragrance dispenser; providing a detector, wherein the detector receives signals from the fragrance dispensers; using a computer, correlating the identifying values transmitted with said signals with the identifying values in the database; displaying the results of the correlation, thereby displaying at least one of the identity of the fragrance devices transmitting said signals and the amount of fragrance in said fragrance devices, thereby providing information on which fragrance dispensers are in need of additional fragrance; and managing said fragrance amounts by providing additional fragrance to said fragrance dispensers in need of additional fragrance.

### Brief Description of the Drawings

Figure 1 shows a dispenser according to one embodiment of the invention.
Figure 2 shows a dispenser according to another embodiment of the invention.
Figure 3 shows a detail of a portion of the dispenser shown in Figure 2.
Figure 4 shows a dispenser according to a further embodiment of the invention.

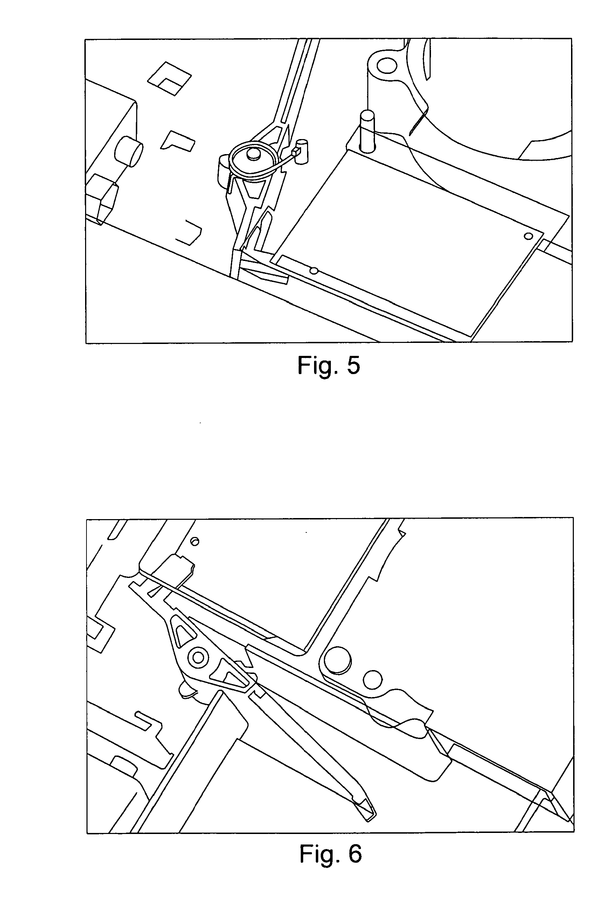

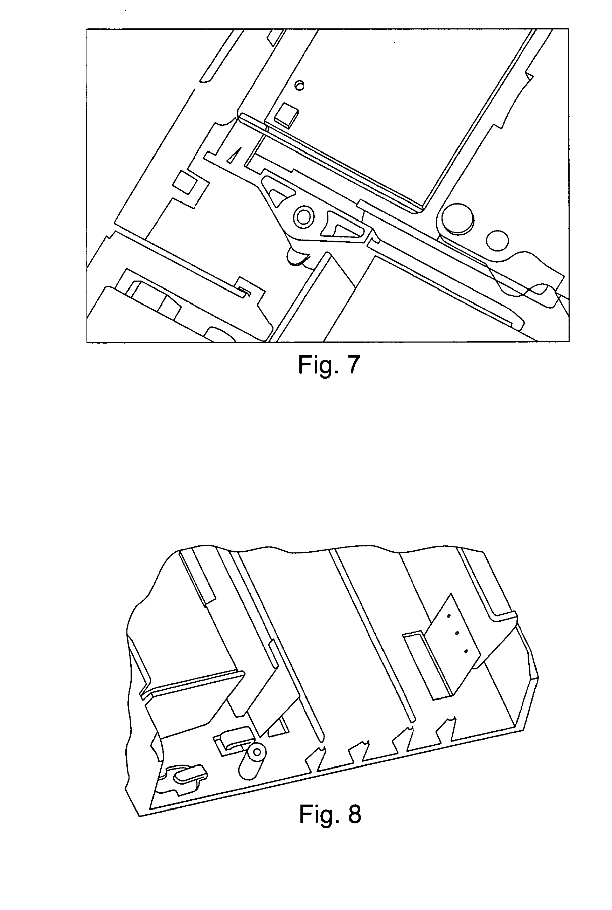

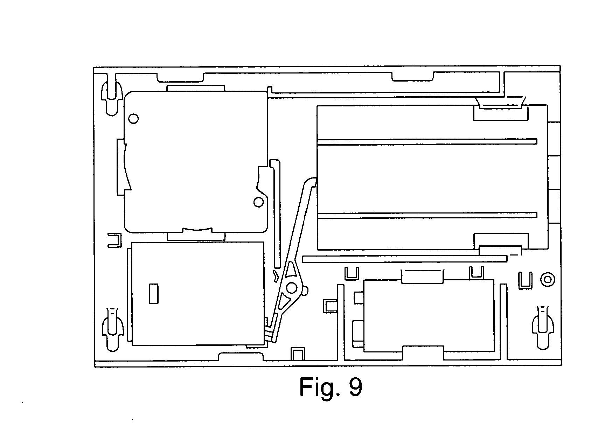

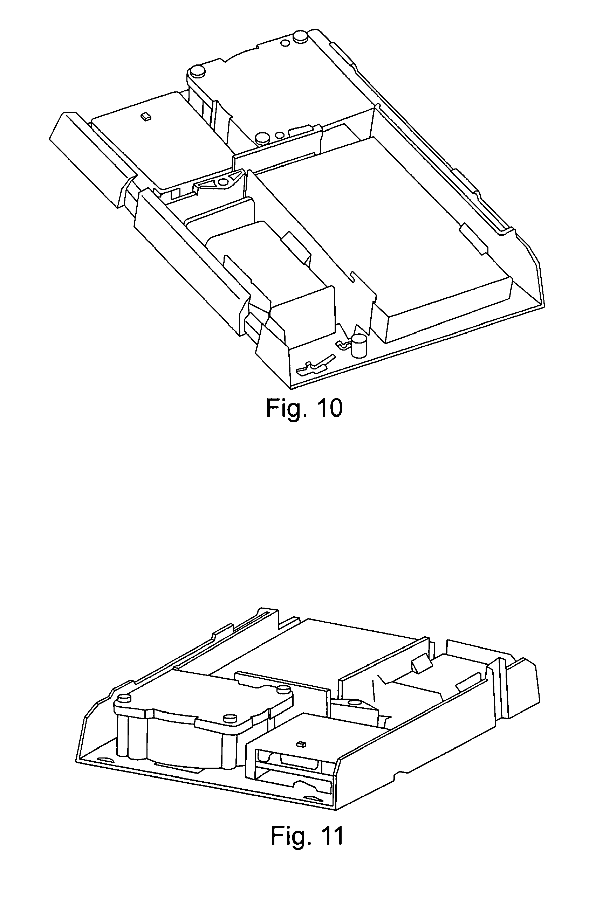

Figures 5-11 show other aspects of interiors of dispensers of embodiments of the invention.

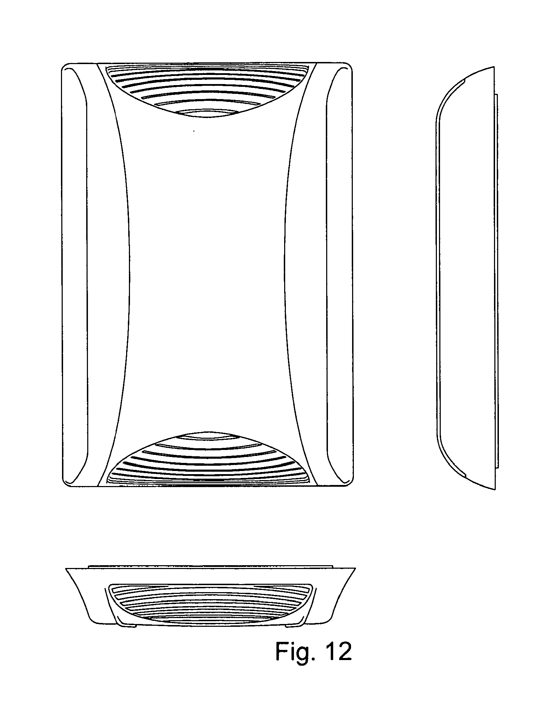

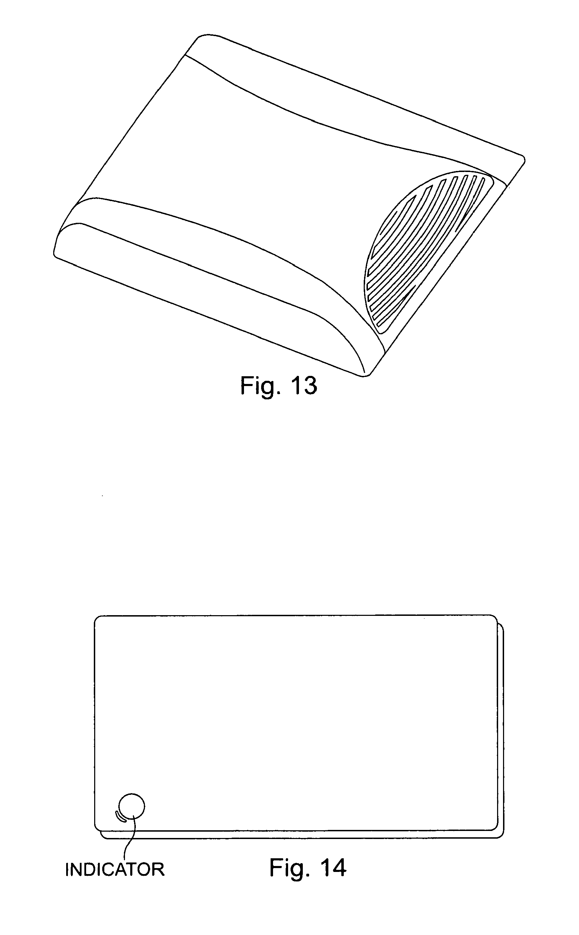

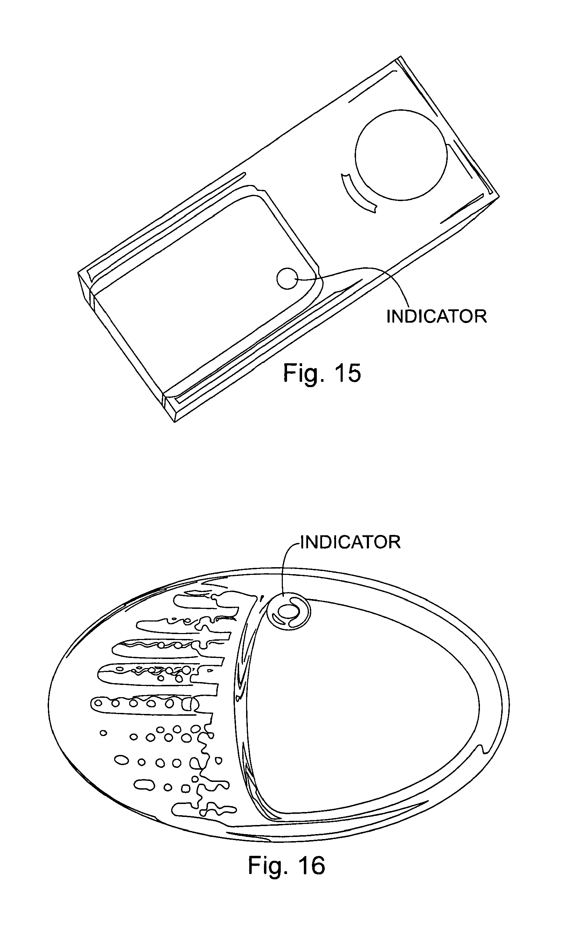

Figures 12-16 show examples of outer casings that may be used in embodiments of the invention. Those skilled in art will recognize that the casing may be a variety of shapes, so long as it allows the fragrance to be released and accommodates the dispenser mechanism.

### Detailed Description of the Invention

Although the invention is described in several aspects and embodiments below, those skilled in the art will, with the benefit of this disclosure, recognize additional aspects, embodiments, and advantages that are within the bounds of the claims.

The invention relates to methods and apparatuses used to notify a user that the contents of a fragrance-dispensing device (hereinafter "dispenser") have exhausted or will soon be exhausted. Features, aspects, and embodiments of the invention will now be discussed with reference to the figures.

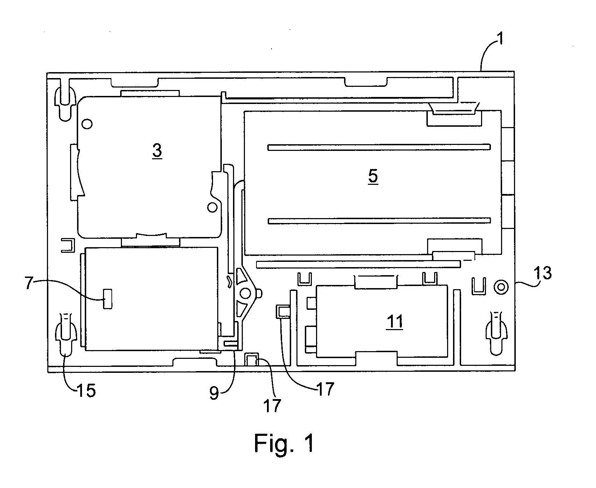

Figure 1 shows a cutaway view of a dispenser according to one embodiment of the invention. In that embodiment, the device includes a casing **1**, which includes a blower **3**, a fragranced pad **5,** a switch **7,** a actuator **9,** a power supply **11,** and a signal **13.**

The casing **1** may have a front side (not shown) and a back side. In a further embodiment of the invention, the casing is unitary and has a slot or other opening for insertion of fragrance. The case may include a door that opens and closes, allowing insertion of fragrance. Other components of the dispenser may be mounted in the casing. In this embodiment the casing includes wall mounts **15.** Any satisfactory artifice may be used to mount the dispenser on a surface, including but not limited to screws, wires, adhesives, tacks, or other items. The casing of this embodiment also includes wire latches **17.** These wire latches allow the casing to be opened, then secured in a closed position without damaging the casing. In other embodiments, the casing may be closed by adhesives or by friction. The casing may include one or more vents.

The embodiment shown in Figure 1 includes a blower **3**. The blower enhances distribution of the fragrance throughout an area. In a further embodiment, the blower is replaced by a heating device that enhances diffusion of fragrance. Other embodiments do not include a blower, heater, or any other device to enhance fragrance distribution. One or more switches 7 may also be included, allowing one to manually activate or deactivate the fragrance dispensation and/or signaling device.

The embodiment shown in Figure 1 further includes a power supply **11**. The power supply may be a battery, or it may be an AC or DC power supply. In one embodiment, the dispenser is wired or plugged into the power supply of the building in which fragrance is to be distributed. The power supply may provide power to the blower, to the signal, or both.

The embodiment of Figure 1 further includes a fragranced pad 5. The fragranced pad contains a fragrance. Fragrances suitable for use in the invention include but are not limited to Cherry Jubilee, Watermelon, Starry Night, Vanilla Breeze, Fresh and Clean, Wildflowers. These fragrances were provided by Belmay, Inc. Other suitable fragrances include, for example, but are not limited to, those listed by the U.S. Food and Drug Administration in Title 21 of the Code of Federal Regulations, Sections 172.510 and 172.515 respectively, which are incorporated by reference herein. Suitable fragrance oils are, for example spice oil, flower oil, and fruit oil. Suitable fragrance chemicals are, for example benzaldehydes, phenols, cinnamic aldehydes and esters thereof, octadienes, dienes, cyclohexadienes, and terpenes.

In one aspect of the invention, the pad is constructed of a polymer. Suitable polymers may be, for example, but are not limited to, high and low density polyethylene, polystyrene, acrylic polymers, polycarbonates, various nylons, and others known to those skilled in the art. Mixtures and copolymers thereof may also be useful in the invention. In a further aspect of the invention, the polymer used is ethyl vinyl acetate ("EVA"). EVA used in the invention may have a molecular weight in the range of, for example, 10,000 Daltons to 100,000 Daltons, more preferably 22,000 to 87,000 Daltons. Fragrance may be introduced into the polymer at weight percents varying from 10 to 90%, from 20 to 80% from 30 to 70%, from 30 to 60%, and from 30 to 50%. In further embodiments, fragrance is introduced into the polymer at a weight percent of about 1%, about 10%, about 20%, about 30%, about 40%, about 50%, about 60%, about 70%, about 80%, about 90%, or about 95%.

The fragrance pad is not limited to any particular size or shape. The shape of the pad may, for example, be a disk (with or without a hole in the center), torus, rectangle, circle or other shape. The pad may include a central slot. Of course, the pad will also have a thickness, and there is no requirement that the thickness be uniform along the length of the pad. Typically the pad will shrink in all dimensions as the fragrance is released, allowing multiple ways to actuate an indicator. For example, a disk or torus may be configured to have a sensor that squeezes together as the interior diameter increases and the exterior diameter decreases.

In a further embodiment, which does not form part of the invention, the pad is replaced by a transparent or translucent container that allows transmission of a signal, for example an infrared or visible light signal. In such an embodiment the fragrance is provided as a liquid in the container. After the amount of liquid has decreased to a predetermined level, the signal is able to travel through the container to a sensor on the opposite side of the container, actuating a signal.

In general, preparation of polymer/fragrance compositions of the invention is facilitated by the use of a solvent. Suitable solvents for use in the invention include but are not limited to isopar M, diisopropyl adipate, isopropyl myristate, benzyl benzoate, propylene glycol and others recognized by those skilled in the art with the benefit of this disclosure.

Those skilled in the art will recognize that a fragranced pad composition included in embodiments of the invention is not limited to inclusion of fragrance, polymer, and a solvent. Various additives may be included to add properties to the mixture or to enhance existing properties. For example, the mixture may include dyes, pigments, substances that improve fragrance absorption/emission, substances that help it keep its shape, help it be formed into a shape, or help it decrease in size uniformly with fragrance release. Both extrusion and injection molding additives suitable for use in the invention will be recognized by those skilled in the art.

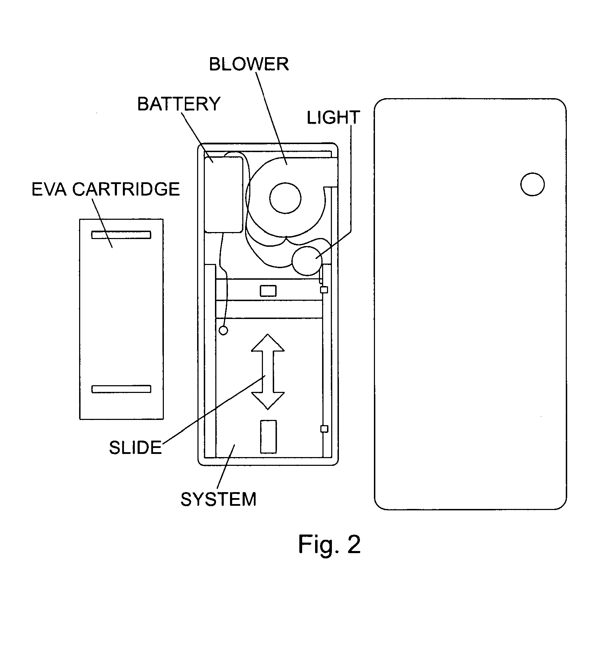

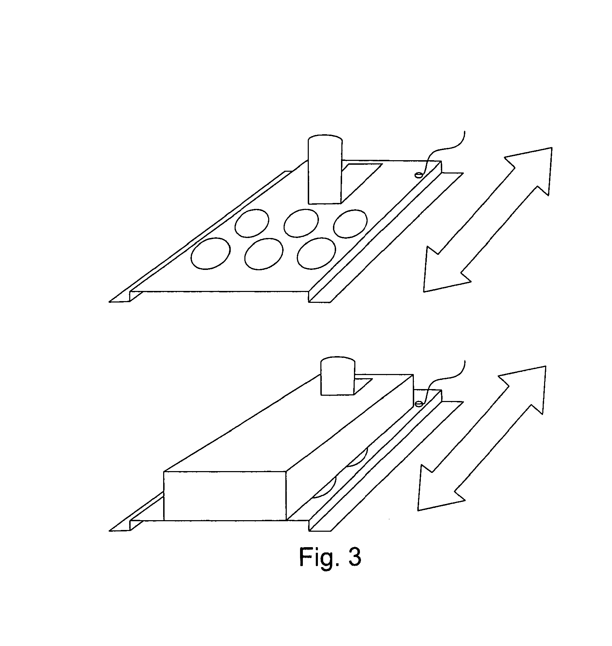

The fragrance oil is absorbed into the polymer. This polymer slowly releases the fragrance in a controlled rate and releases most of the fragrance. As the polymer releases the fragrance, the fragranced pad shrinks. In one embodiment of the invention, shown in Figure 2 and in detail in Figure 3, the fragranced pad is placed in a tray that slides as the fragrance is exhausted.

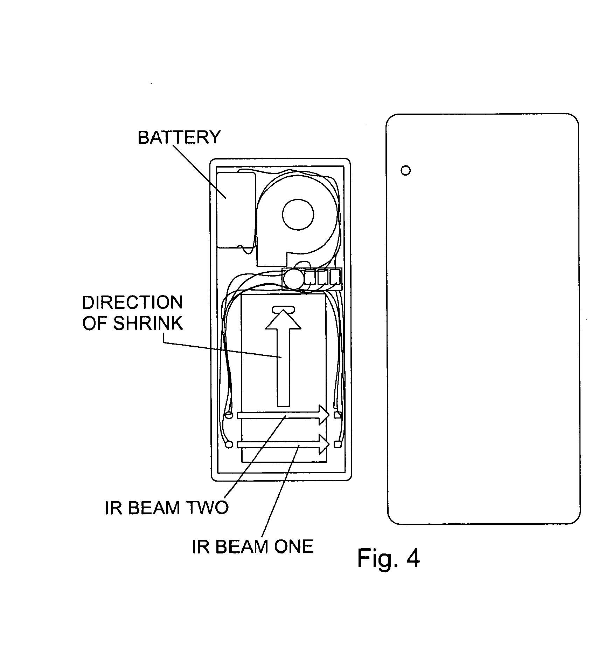

When the fragranced pad shrinks beneath a predetermined size, an actuator 9 actuates a signal 13. In the embodiment shown in Figure 1, the actuator is a spring-loaded arm that gradually moves as the fragranced pad shrinks, eventually completing a circuit. In the embodiment of Figure 1, the signal is an LED. In other embodiments of the invention, the actuator is a button that is depressed until the fragranced pad shrinks below a predetermined size. The actuator may also be a infrared signal, as shown in Figure 4, that is allowed to register after the fragranced pad has shrunk below a predetermined size. The infrared actuator is particularly useful in embodiments in which the fragrance is contained in a liquid carrier, the level of which decreases as the amount of fragrance decreases. An actuator may also be a tensioned spring, either linear or circular, that is allowed to expand as the amount of polymer decreases.

A dispenser may include more than one actuator, which allows it to generate more than one signal. This allows the dispenser to signal, for instance, when the amount of fragrance had decreased to less than half the original amount, and then signal again when the fragrance is exhausted. Figure 4, for example, shows a plurality of infrared actuators.

A dispenser may include an sensor that continuously determines the amount of fragrance that remains in the dispenser. This information (obtained, for example, as a function of the decreasing size of the polymer pad fragrance carrier) may be continuously or intermittently relayed to th

The signal may take multiple forms. It may be visual, such as a light or colored tab. It may be an audio signal, such as a buzz or beep. It may be a signal that is sent to a remote location by wifi, bluetooth, radio, or other electromagnetic form. The signal may be sent to a central processing device, such as a computer, which allows coordination of replacement of the fragrance. The signal may be detected by a handheld device carried by a maintenance technician. A single dispenser may emit more than one signal when the actuator has engaged. For example, when the amount of fragrance has decreased to a predetermined level, both an audio signal and a visual signal may be produced. A visual signal (such as, for example, an LED signal) may be configured to light continuously when the fragrance is at one level, then blink after the amount of fragrance has decreased to a lower level.

The attached Table 1 shows an example of shrinkage of a fragrance pad that is 40% fragrance and 60% ethyl vinyl acetate. This demonstrates that the EVA polymer may shrink in a uniform manner as the fragrance is released, allowing the sensor to be actuated.

## Claims

1. A fragrance dispenser comprising:
a) a fragrance, said fragrance being impregnated within a polymer carrier, said polymer carrier decreasing in size as said fragrance is released;
b) a sensor for determining the amount of said polymer carrier, said sensor being oriented such that said sensor is actuated when at least one physical dimension of said carrier falls below a predetermined value;
c) a signaling device for displaying information related to the amount of said polymer carrier as an amount of fragrance, wherein said signaling device is operatively connected to and actuated by said sensor.

2. The fragrance dispenser of claim 1, wherein said polymer carrier is polyethylene-vinyl acetate.

3. The fragrance dispenser of claim 1, further comprising a second sensor, wherein said second sensor is operatively connected to a second signaling device, and wherein said second sensor indicates a second predetermined amount of said polymer carrier.

4. The fragrance dispenser of claim 1, wherein said signaling device continuously broadcasts information related to the amount of said fragrance.

5. The fragrance dispenser of claim 1, wherein said signaling device transmits a signal selected from the group consisting of a radio signal, a Bluetooth signal, an electrical signal, a wi-fi signal, a light, a sound, a switch, and combinations thereof.

6. A system for managing the fragrance amounts in a plurality of fragrance dispensers, comprising:
a) providing a plurality of fragrance dispensers according to claim 1;
b) assigning each fragrance dispenser of step (a) a separate identifying value that is transmitted with a signal being transmitted by said signaling device, said identifying value indicating the amount of said polymer carrier as an amount of fragrance;
c) maintaining a database of said identifying values;
d) providing a detector, wherein said detector receives signals from said fragrance dispensers;
e) using a computer, correlating the identifying values transmitted with said signals with the identifying values in said database;
f) displaying the results of said correlation, thereby displaying at least one of the identity of the fragrance devices transmitting said signals and the amount of fragrance in said fragrance devices, thereby providing information on which fragrance dispensers are in need of additional fragrance; and
g) managing said fragrance amounts by providing additional fragrance to said fragrance dispensers in need of additional fragrance.

7. A method for notifying a user that the amount of fragrance in a fragrance dispenser has reached a predetermined level, comprising:
a) providing a fragrance dispenser according to any one of claims 1-5;
b) dispensing fragrance from said fragrance dispenser until said sensor actuates said signal, wherein said signal notifies a user of the amount of fragrance in said dispenser.

8. Use of a fragrance dispenser according to any one of claims 1-5 for notifying a user that the amount of fragrance in the fragrance dispenser has reached a predetermined level, wherein fragrance is dispensed from said fragrance dispenser until the sensor actuates the signal, and wherein said signal notifies the user of the amount of fragrance in said dispenser.

## Patentansprüche

1. Ein Duftstoffspender umfassend:
a) einen Duftstoff, wobei besagter Duftstoff innerhalb eines Polymerträger imprägniert wird, wobei besagter Polymerträger seine Größe verringert, während besagter Duftstoff freigesetzt wird
b) einen Sensor zum Bestimmen des Umfanges des besagten Polymerträgers, wobei der Sensor in der Art gestaltet ist, dass der Sensor ausgelöst wird, wenn mindestens eine physikalische Größe des Trägers unter einem vorgegebenen Wert fällt.
c) Eine Signalvorrichtung zur Darstellung von Informationen, die in Zusammenhang mit dem Umfang des besagten Polymerträgers und der Menge des Duftstoffes stehen, wobei besagte Signalvorrichtung funktionsfähig mit besagtem Sensor verbunden ist und durch besagten Sensor ausgelöst wird.

2. Der Duftstoffspender nach Anspruch 1, wobei besagter Polymerträger Polyethylen-Vinylacetat ist.

3. Der Duftstoffspender nach Anspruch 1, ferner umfassend einen zweiten Sensor, wobei besagter zweiter Sensor mit einer zweiten Signalvorrichtung funktionsfähig verbunden ist und wobei besagter zweiter Sensor einen zweiten vorgegebenen Umfang des besagten Polymerträgers anzeigt.

4. Der Duftstoffspender nach Anspruch 1, wobei besagte Signalvorrichtung kontinuierlich Informationen bezüglich der Menge des besagten Duftstoffes überträgt.

5. Der Duftstoffspender nach Anspruch 1, wobei besagte Signalvorrichtung ein Signal aus der Gruppe bestehend aus einem Radiosignal, einem Bluetooth Signal, einem elektrischen Signal, einem Wi-Fi Signal, einem Licht Signal, einem Ton Signal, einem Schaltsignal und Kombinationen daraus überträgt.

6. Ein System zum Überwachen der Duftstoffmenge in einer Vielzahl von Duftstoffspendern, umfassend:
a) Bereitstellung einer Vielzahl von Duftstoffspendern gemäß Anspruch 1;
b) Zuordnung eines separaten Identifikationswertes zu jedem einzelnen Duftstoffspender von Schritt a), welcher mittels eines Signals durch besagte Signalvorrichtung übermittelt wird, besagter Identifikationswert gibt den Umfang des besagten Polymerträgers als Menge des Duftstoffes an
c) Führen einer Datenbank mit besagten Identifikationswerten
d) Bereitstellen eines Detektors, wobei besagter Detektor Signale von besagten Duftstoffspendern erhält
e) Verwenden eines Computers, der die mit besagten Signalen übermittelten Identifikationswerte den Identifikationswerten aus besagter Datenbank zuordnet
f) Anzeige der Ergebnisse von besagter Zuordnung, wobei mindestens eine der durch besagte Signale übermittelten Identitäten der Duftstoffvorrichtungen und die Menge des Duftstoffes in besagten Duftstoffvorrichtungen angezeigt wird, wobei Informationen geliefert werden, welche der Duftstoffspender zusätzlichen Duftstoff benötigen, und
g) Verwalten der Duftstoffmengen durch die Versorgung von besagten Duftstoffvorrichtungen mit zusätzlichen Duftstoffen, die zusätzlichen Duftstoff benötigen

7. Ein Verfahren zum Benachrichtigen eines Benutzers darüber, dass die Duftstoffmenge in der Duftstoffvorrichtung einen vorbestimmten Füllstand erreicht hat, umfassend
a) Bereitstellung eines Duftstoffspenders gemäß einem der Ansprüche 1-5
b) Duftstoffabgabe von besagtem Duftstoffspender, bis besagter Sensor das besagte Signal auslöst, wobei besagtes Signal einem Benutzer die Duftstoffmenge in besagtem Duftstoffspender anzeigt

8. Eine Verwendung eines Duftstoffspenders gemäß einem der Ansprüche 1-5, um einem Benutzer anzuzeigen, dass die Duftstoffmenge in dem Duftstoffspender einen vorbestimmten Füllstand erreicht hat, wobei der Duftstoff solange vom Duftstoffspender abgegeben wird, bis der Sensor ein Signal auslöst und wobei besagtes Signal dem Benutzer die Duftstoffmenge im besagten Spender anzeigt.

## Revendications

1. Un diffuseur de parfum comprenant :
a) un parfum, ledit parfum étant imprégné dans un support polymère, ledit support polymère diminuant en taille au fur et à mesure que ledit parfum est libéré ;
b) un capteur pour déterminer le volume dudit support polymère, ledit capteur étant orienté de telle manière à ce que ledit capteur soit activé lorsqu'au moins une dimension physique dudit support chute en dessous d'une valeur prédéfinie ;
c) un dispositif de signalisation affichant les informations relatives au volume dudit support polymère comme quantité de parfum, ledit dispositif de signalisation étant fonctionnellement connecté et activé par ledit capteur.

2. Le diffuseur de parfum de la revendication n°1, ledit support polymère étant en polyéthylène-acétate de vinyle.

3. Le diffuseur de parfum de la revendication n°1, comprenant également un second capteur, ledit second capteur étant fonctionnellement connecté à un second dispositif de signalisation et ledit second capteur indiquant une seconde quantité prédéfinie dudit support polymère.

4. Le diffuseur de parfum de la revendication n°1, ledit dispositif de signalisation diffusant continuellement des informations relatives à la quantité dudit parfum.

5. Le diffuseur de parfum de la revendication n°1, ledit dispositif de signalisation transmettant un signal sélectionné à partir du groupe comprenant un signal radio, un signal Bluetooth, un signal électrique, un signal wi-fi, un éclairage, un son, un interrupteur et des combinaisons de ces derniers.

6. Un système pour gérer les quantités de parfum dans plusieurs diffuseurs de parfum, comprenant :
a) la fourniture de plusieurs diffuseurs de parfum conformément à la revendication n°1 ;
b) l'affectation, à chaque diffuseur de parfum de l'étape (a), d'une valeur d'identification distincte qui est transmise avec un signal transmis par ledit dispositif de signalisation, ladite valeur d'identification indiquant le volume dudit support polymère comme quantité de parfum ;
c) la gestion d'une base de données desdites valeurs d'identification ;
d) la fourniture d'un détecteur, ledit détecteur recevant des signaux desdits diffuseurs de parfum ;
e) l'utilisation d'un ordinateur en corrélant les valeurs d'identification transmises avec lesdits signaux avec les valeurs d'identification dans ladite base de données ;
f) l'affichage des résultats de ladite corrélation, montrant ainsi au moins l'une des identités des diffuseurs de parfum transmettant lesdits signaux et la quantité de parfum dans lesdits diffuseurs de parfum, fournissant ainsi des informations sur les diffuseurs de parfum qui ont besoin de plus de parfum et
g) la gestion desdites quantités de parfum en fournissant plus de parfum auxdits diffuseurs de parfum qui ont besoin de plus de parfum.

7. Une méthode pour notifier à un utilisateur que la quantité de parfum dans un diffuseur de parfum a atteint un niveau prédéfini, comprenant :
a) la fourniture d'un diffuseur de parfum conformément à l'une des revendications n°1 à n°5 ;
b) la diffusion d'un parfum à partir du dit diffuseur de parfum jusqu'à ce que ledit capteur active un dit signal, ledit signal notifiant à un utilisateur la quantité de parfum dans ledit diffuseur.

8. L'utilisation d'un diffuseur de parfum conformément à l'une des revendications n°1 à n°5 pour notifier à un utilisateur que la quantité de parfum dans le diffuseur de parfum a atteint un niveau prédéfini, le parfum étant diffusé à partir dudit diffuseur de parfum jusqu'à ce que le capteur active le signal et ledit signal notifiant à l'utilisateur la quantité de parfum dans ledit diffuseur.
